# EUROPEAN PATENT APPLICATION

(11) **EP 2 624 004 A1**
(43) Date of publication of application: **07.08.2013**
(21) Application number: 12153983.7
(22) Date of filing: 06.02.2012
(51) Int. Cl.: G01R 33/48

(54) **Temperature determination using magnetic resonance B1 field mapping**

(71) Applicant: Koninklijke Philips Electronics N.V., 5621 BA Eindhoven (NL); Philips Intellectual Property & Standards GmbH, 20099 Hamburg (DE)
(72) Inventor: Leussler, Christoph, 5600 AE Eindhoven (NL); Katscher, Ulrich, 5600 AE Eindhoven (NL)
(74) Representative: Damen, Daniel Martijn

(57) **Abstract**

A medical apparatus (600, 700, 800, 900, 1000) comprising a magnetic resonance imaging system (602) comprising a magnet (604) with an imaging zone (608) for acquiring magnetic resonance data (642, 748) from a subject (618) from within the imaging zone. The medical apparatus further comprises a memory (632) for storing machine executable instructions (660, 662, 664, 760, 762, 764). The medical apparatus further comprises a processor (626) for controlling the medical apparatus. Execution of the instructions causes the processor to: acquire (100, 200, 300, 400, 506) B1 field map magnetic resonance data (642) using the magnetic resonance imaging system and determine (102, 206, 306, 408, 512) a temperature map (646) using the B1 field map magnetic resonance data.

## Description

### TECHNICAL FIELD

The invention relates to measurement of temperature using magnetic resonance imaging, in particular to using measurement of the B 1 magnetic field for temperature measurement.

### BACKGROUND OF THE INVENTION

Magnetic resonance thermometry may be used to determine either the absolute temperature of a volume or a change in temperature, depending upon the technique used. For determining the absolute temperature several magnetic resonance peaks are typically measured. Methods which measure changes in temperature are typically faster and have been used to take temperature measurements for guiding thermal treatments. For example Proton resonance frequency shift based MR thermometry may be employed to provide temperature maps in water inside the tissue during the ablation procedure for real-time feedback control of the heating process.

In high-intensity focused ultrasound (HIFU) therapy, reliable real-time temperature monitoring using e.g. Magnetic Resonance Imaging (MRI) is necessary to ensure a sufficient thermal necrosis to the target while avoiding excessive heating and damage of surrounding healthy tissues. To achieve sufficient temporal and spatial resolution, fast imaging is required preferably with a high spatial resolution while maintaining a sufficient SNR for reconstruction of reliable temperature measurements.

PCT patent application WO 2007/017779 A2 describes a system where the electric permittivity and/or conductivity distribution is computed using the magnetic induction field strength.

### SUMMARY OF THE INVENTION

The invention provides for a medical apparatus, a method of operating a medical apparatus and a computer program product in the independent claims. Embodiments are given in the dependent claims.

Embodiments of the invention may provide a rapid and accurate means of measuring temperature using magnetic resonance imaging. The method uses electrical properties thermography and the temperature distribution is derived from local electrical properties, such as the local electrical conductivity and/or permittivity. The conductivity and/or permittivity may be temperature dependent.

Embodiments of the invention may provide for a non-invasive thermography method that is suitable for feedback control of a MR image guided hyperthermia or hypothermia therapy method.

The conductivity of tissue depends on the applied frequency and the temperature. Relative changes of conductivity of a lesion during treatment with RF energy depend on biochemical changes and on tissue temperature. Temperature-dependant conductivity and/or permittivity changes may be used to determine the temperature of tissues with MRI.

Thermal energy may be used to treat tumors in such organs as the liver and kidney. Radiofrequency (RF) ablation, microwave ablation, and hyperthermia therapy use the penetration and absorption of electromagnetic waves, which depend on tissue conductivity. The conductivity and/or permittivity of tissue depend on frequency and temperature. Relative changes of conductivity of the lesion during treatment with RF energy depend on biochemical changes and on tissue temperature. Tissue conductivity is an important parameter during online treatment using local RF energy absorption. While conductivity as a function of frequency is well documented, temperature effects of conductivity during treatment can be measured using MR-based Electric Properties Tomography (EPT). EPT provides a non-invasive means to assess electric tissue properties such as conductivity. It is based on the measurement and post-processing of the complex active component of the RF excitation field (B1).

A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network.

'Computer memory' or 'memory' is an example of a computer-readable storage medium. Computer memory is any memory which is directly accessible to a processor. Examples of computer memory include, but are not limited to: RAM memory, registers, and register files.

'Computer storage' or 'storage' is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

A 'processor' as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. Many programs have their instructions performed by multiple processors that may be within the same computing device or which may even be distributed across multiple computing devices.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

Magnetic resonance data may comprise the measurements of radio frequency signals emitted by atomic spins by the antenna of a Magnetic resonance apparatus during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonance frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonance frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

Spectroscopic magnetic resonance data is defined herein as being the recorded measurements of radio frequency signals emitted by atomic spins by the antenna of a Magnetic resonance apparatus during a magnetic resonance imaging scan which contains information which is descriptive of multiple resonance peaks.

The spectroscopic magnetic resonance data may for instance be used to perform a proton spectroscopic (PS) imaging based temperature mapping method which can produce temperature maps on absolute scale. This absolute scale temperature map may therefore be used to perform a temperature calibration. This method relies on the physical principles of water proton resonance shift temperature dependence as the proton resonance frequency method, but the acquisition method is different: the frequency shift is calculated from the magnetic resonance spectra. The shift is calculated from the position difference of the water and a reference proton peak. Protons in lipids may for example be used as reference, as their resonance frequency is known to be almost independent of temperature, while the water proton peak has linear dependence on temperature. This can be done in the voxels, where both tissue types are present. If water and lipids do not exist in the same voxel, one may try to use some other tissue type than lipids as reference. If not successful, there may be some voxels where the reference peaks, and therefore the temperature data, are not available. Interpolation and/or temperature filtering may be used to help these situations, since body temperature is normally not expected to change rapidly spatially with the highly localized temperature rise typically caused by thermal therapy being an obvious exception. The utilization of reference peaks makes the method relatively independent of field drifts or inter-scan motion. Because the scanning takes a time of at least on the order of one minute with current methods, the PS method is susceptible to intra-scan motion or temperature change during scanning. In a case where temperature is constant or temperature variation is small both in time and space, the method is able to produce useful information. For example, with the Magnetic Resonance Guided High Intensity Focused Ultrasound (MR-HIFU), the PS method can be used to provide the actual body temperature distribution before start of MR-HIFU or other temperature treatment as opposed to using a spatially homogeneous starting temperature taken as the body core temperature measured with a thermometer probe. Alternatively, the PS method can be used as a sanity check for cumulated temperature between treatment heatings outside treatment area.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

In one aspect the invention provides for a medical apparatus comprising a magnetic resonance imaging system for acquiring magnetic resonance data from the subject within an imaging zone of a magnet. The magnetic resonance imaging system comprises the magnet and the magnet has an imaging zone. The medical apparatus further comprises a memory for storing machine executable instructions. The medical apparatus further comprises a processor for controlling the medical apparatus. That is the processor is capable of controlling the magnetic resonance imaging system. Execution of the instructions causes the processor to acquire B1 field map magnetic resonance data using the magnetic resonance imaging system. B 1 field map magnetic resonance data as used herein encompasses magnetic resonance data. The B 1 field map magnetic resonance data comprises data or information which may be used to construct a B 1 field map. The B 1 field map magnetic resonance data may be acquired in a variety of ways.

Methods of acquiring a B 1 field amplitude map are well known and any of these methods may be used. Examples of acquiring a B 1 field amplitude map include, but are not limited to: a based on a magnetisation preparation Turbo FLASH sequence, the double-angle method which uses the ratio of two images acquired with different flip angles, measurement of the effect caused by a saturation pre-pulse in turbo field echo (TFE) pulse sequence, a method based on the TESSA (Transition from Equilibrium into Steady State Acquisition) principle for simultaneous in vivo quantitation of B 1 amplitude and T1 maps.

Some methods of acquiring a B1 field phase map are published and any of these methods may be used. The B 1 field phase map is based on a phase map of a standard MR image, which however preferably do not contain comtributions from off-resonance effects like B0 inhomogeneities and susceptibility. Thus, it is recommended to use, for example, phase maps of one of the following magnetic resonance sequence types: spin-echo, turbo spin-echo, balanced fast field echo, steady state free precision. These phase maps contain contributions from both, B1 field (i.e., RF transmission) and RF reception, therefore they are typically called transceive phase map. Since it is typically assumed that these two contributions are of equal size, an estimation of the B 1 field phase map can be obtained by dividing the transceive phase map by two.

Execution of the instructions further cause the processor to determine a temperature map using the B1 field map magnetic resonance data. This embodiment may be beneficial because the B 1 field map data that is acquired may be used to determine a temperature which may be used or displayed and may be of use to a physician. The temperature map may also be used to control a heating system.

In some embodiments the temperature map and any B 1 field map magnetic resonance mapping may be spatially dependent.

In some embodiments the temperature map may be determined by calculating a temperature change from an initial measurement. In other embodiments a lookup table may be used for a conductivity and/or permittivity which is calculated from a B 1 field map.

In another embodiment execution of the instructions further cause the processor to determine a B 1 field phase map using the B 1 field map magnetic resonance data. The B1 field phase map as used herein is just the phase component of a B 1 field mapping or map. Execution of the instructions further cause the processor to calculate a conductivity map from the B 1 field phase map. The temperature map is determined at least partially using the conductivity map. In this embodiment the conductivity may be approximated by using only the phase component of a B 1 field map. The calculation of an approximate conductivity map enables the calculation of an approximate temperature map. This embodiment may be beneficial because it may be faster to just determine the B 1 field phase map than determining both the B1 field phase and amplitude. This may lead to a more rapid determination of the temperature.

A method of determining an approximate conductivity map using B 1 phase information was described in T. Voigt et. al., "In vivo quantitative conductivity imaging based on B 1 phase information," In Proceedings of the 18th Annual Meeting of ISMRM, p. 2865,2010.

In some embodiments the conductivity map and resulting temperature map may be spatially dependent. Determining the B 1 field phase map may include simply taking the phase of the field map.

In another embodiment execution of the instructions further cause the processor to determine a B 1 amplitude map from the B 1 field map magnetic resonance data. Execution of the instructions further cause the processor to calculate a permittivity map from the B 1 field amplitude map. The temperature map is determined at least partially using the permittivity map. This embodiment may also be beneficial because only the amplitude of the B1 map is used to calculate the permittivity. This permittivity map is an approximate permittivity map which enables the calculation of an approximate temperature map. This may enable the more rapid determination of the temperature map. In some embodiments the permittivity map and the resulting temperature map may be spatially dependent.

A method of determining an approximate permittivity map using B 1 amplitude information was described in Katscher et. al., "Permittivity determination via phantom and in vivo B 1 mapping," In Proceedings of the 18th Annual Meeting of ISMRM, p. 239, 2010. ISMRM 18 (2010) 239.

In another embodiment execution of the instructions further cause the processor to determine a B 1 amplitude map and a B 1 phase map using the B1 field map magnetic resonance data. The combination of the B 1 amplitude map and the B 1 phase map is a full B 1 field mapping or map. Execution of the instructions further cause the processor to calculate a conductivity map from the B 1 field phase map and the B 1 field amplitude map. Execution of the instructions further cause the processor to calculate a permittivity map from the B1 field phase map and the B 1 field amplitude map. The temperature map is determined at least partially using the conductivity and the permittivity map. In this embodiment the conductivity is determined from both the phase and the amplitude. The permittivity is also calculated from both the phase and the amplitude. Both the conductivity and the permittivity are determined more accurately. The combination of using both the conductivity and the permittivity may enable the more accurate determination of the temperature map.

In some embodiments the temperature map is only determined using a conductivity map calculated using the B 1 field phase map and the B 1 field amplitude map.

In another embodiment the temperature map is determined using only the permittivity map calculated from the B 1 field phase map and the B1 field amplitude map.

In another embodiment execution of the instructions further cause the processor to acquire magnetic resonance data using the magnetic resonance imaging system. Execution of the instructions further causes the processor to reconstruct image data from the magnetic resonance data. In some instances the magnetic resonance data may be identical with the B 1 field map magnetic resonance data. That is to say that they are identical or they may be acquired at the same time. This embodiment may be advantageous because it may be beneficial to acquire image data at the same time that a temperature map is determined.

In another embodiment execution of the instructions further cause the processor to segment the image data. The processor may use a standard image segmentation module or technique to segment the image. Segmenting the image data as used herein encompasses dividing the image into different regions of tissue type or organs. Execution of the instructions further cause the processor to determine a tissue type map using the segmented image data. The determination of the temperature map is calibrated at least partially using the tissue type map. This embodiment may be beneficial because if the tissue type is known then the conductivity and/or permittivity of a particular type may have its temperature dependence known. This may enable an absolute calibration method of determining a temperature map using the B 1 field map magnetic resonance data.

Image segmentation may be done using an image segmentation module. The image segmentation may be spatially dependent.

In another embodiment the medical apparatus further comprises a display. The method further comprises the step of displaying the temperature map and the image data on the display. This embodiment may be beneficial because the displaying of the temperature map and the image data on the display may enable more accurate or better interpretation of the temperature map and/or the image data. In some embodiments the temperature map may be superimposed on the image data to enable better interpretation of the temperature map and/or the image data.

In another embodiment execution of the instructions further cause the processor to estimate a specific absorption ratio of electromagnetic energy caused by the acquisition of the magnetic resonance data using the temperature map. The specific absorption ratio may be useful when performing magnetic resonance imaging scans on a subject. When performing magnetic resonance imaging, an antenna or coil is used to generate the B 1 field or excitation field during the process of performing the magnetic resonance imaging. These radio waves or B 1 field may cause radio frequency heating of portions of the subj ect. Particularly as the magnetic field strength is increased the frequency of the B 1 field is also increased. If too high a power for the excitation field is used it may lead to heating of portions of the subject which may be harmful or dangerous. Estimating the specific absorption ratio may enable the safer acquisition of magnetic resonance data.

In another embodiment execution of the instructions further cause the processor to acquire spectroscopic magnetic resonance data. Execution of the instructions further causes the processor to calculate a calibration thermal map using the spectroscopic magnetic resonance data. Determination of the temperature map is calibrated at least partially using the calibration thermal map. This embodiment may be particularly beneficial because acquiring spectroscopic magnetic resonance data may enable the absolute determination of temperature. The calibration thermal map may be used as a baseline and then the method of determining a temperature map using the B1 field map magnetic resonance data may be used to measure the temperature change with respect to the calibration thermal map. This may enable the accurate determination of temperature within a subject.

In another embodiment the calculation of the temperature map is calibrated at least partially using an assumed calibration thermal map. Before any sort of magnetic resonance imaging data or heating of or cooling of the subj ect is performed, it may be possible to generate a reasonable calibration by assuming the temperature of the subject. This may be performed by assuming a uniform temperature within the subject or by using a bubble of temperature distribution within a subject which is representative of normal physiology.

In another embodiment the medical apparatus further comprises a heating system. A heating system as used herein encompasses a system for locally heating a portion of the subject. The processor may be used for controlling the heating system also. Execution of the instructions further cause the processor to receive a treatment plan descriptive of the heating of a target zone within the subject. A treatment plan as used herein may encompasses a plan or machine controls developed by a physician or other medical professional which are descriptive of the controls that can be used to generate such controls to control the heating system to heat a target zone within the subject.

Execution of the instructions further causes the processor to heat the target zone using the heating system. The heating system is controlled in accordance with the treatment plan and the temperature map. The temperature map may be used to help generate controls which are used such that the treatment plan may be followed. The temperature map may also be used to help protect critical zones within the subject that may be adjacent to the target zone or inadvertently heated by the heating system. For instance when the target zone is heated it may be desirable to keep tissue surrounding the target zone below a predetermined temperature. This may be used to monitor the process of the heating of the target zone to stop the heating system to prevent it from heating the adjacent regions. The temperature map may also be used to ensure that a region of the subject is not unintentionally heated, for instance when performing high-intensity focused ultrasound portions of the near field may be inadvertently heated.

In another embodiment execution of the instructions further cause the processor to repeatedly reacquire the B 1 field map magnetic resonance data and recalculate the temperature map. Execution of the instructions further cause the processor to adjust the heating of the target zone using the recalculated temperature map. This embodiment may be beneficial because the temperature map is used to form a control loop for more accurately controlling the heating system.

This embodiment could also include repeatedly determining the B 1 field amplitude and/or phase map.

In another embodiment the heating system is a high-intensity focused ultrasound system.

In another embodiment the heating system is a radio-frequency heating system.

In another embodiment the heating system is a microwave ablation system.

In another embodiment the heating system is a hyperthermia therapy system.

In another embodiment the heating system is a laser ablation system.

In another embodiment the heating system is an infrared ablation system.

In another aspect the invention provides for a method of determining a temperature map (646). The method comprises the step of acquiring (100, 200, 300, 400, 506) B 1 field map magnetic resonance data (642) using a magnetic resonance imaging system. The method further comprises the step of determining (102, 206, 306, 408, 512) the temperature map (646) using the B1 field map magnetic resonance data.

In another aspect the invention provides for a method of operating a medical apparatus comprising a magnetic resonance imaging system. The magnetic resonance imaging system comprises a magnet with an imaging zone for acquiring magnetic resonance data from the subject within the imaging zone. The method comprises the step of acquiring B 1 field map magnetic resonance data using the magnetic resonance imaging system. The method further comprises the step of determining a temperature map using the B 1 field map magnetic resonance data.

In another aspect the invention provides for a computer program product containing computer executable code for execution by a processor controlling a medical apparatus. The computer program product may for instance be stored on a non-transitory computer-readable medium. The medical apparatus comprises a magnetic resonance imaging system. The magnetic resonance imaging system comprises a magnet with an imaging zone for acquiring magnetic resonance data from a subject. The subject is within the imaging zone. Execution of the instructions causes the processor to acquire B 1 field map magnetic resonance data using the magnetic resonance imaging system. Execution of the instructions further cause the processor to determine a temperature map using the B 1 field map magnetic resonance data.

### BRIEF DESCRIPTION OF THE DRAWINGS

In the following preferred embodiments of the invention will be described, by way of example only, and with reference to the drawings in which:
Fig. 1 shows a flow diagram which illustrates a method according to an embodiment of the invention;
Fig. 2 shows a flow diagram which illustrates a method according to a further embodiment of the invention;
Fig. 3 shows a flow diagram which illustrates a method according to a further embodiment of the invention;
Fig. 4 shows a flow diagram which illustrates a method according to a further embodiment of the invention;
Fig. 5 shows a flow diagram which illustrates a method according to a further embodiment of the invention; Fig. 6 illustrates a medical apparatus according to an embodiment of the invention;
Fig. 7 illustrates a medical apparatus according to an embodiment of the invention;
Fig. 8 illustrates a medical apparatus according to an embodiment of the invention;
Fig. 9 illustrates a medical apparatus according to an embodiment of the invention;
Fig. 10 illustrates a medical apparatus according to an embodiment of the invention;
Fig. 11 shows a plot of the temperature versus the normalized conductivity of a variety of different samples which are measured using electric properties tomography; and
Fig. 12 shows a plot of the temperature versus the normalized permittivity of a variety of different samples which are measured using electric properties tomography.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

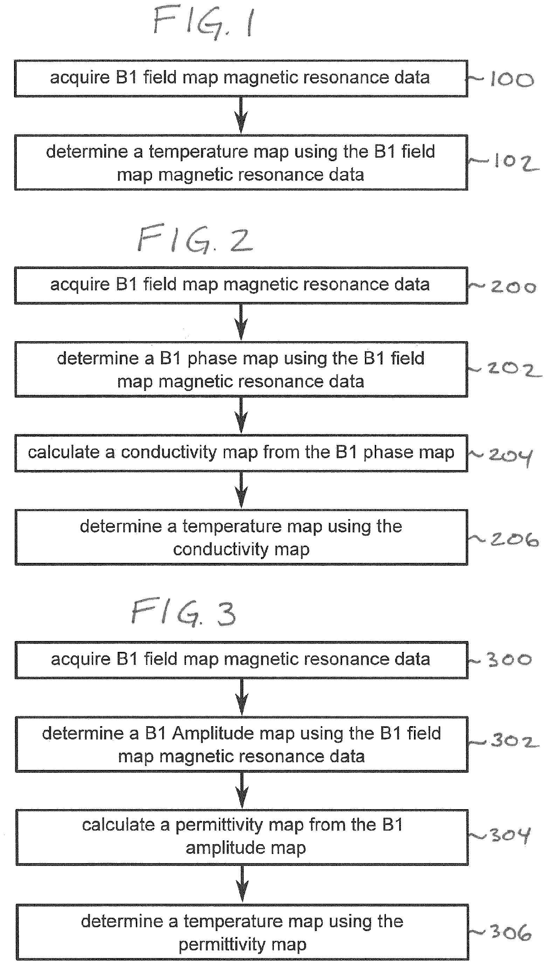

Fig. 1 shows a flow diagram which illustrates a method according to an embodiment of the invention. In step 100 B 1 field map magnetic resonance data is acquired. Next in step 102 a temperature map is determined using the B 1 field map magnetic resonance data.

Fig. 2 shows a flow diagram which illustrates a method according to a further embodiment of the invention. In step 200 B1 field map magnetic resonance data is acquired using the magnetic resonance imaging system. Next in step 202 a B 1 phase map is determined using the B 1 field map magnetic resonance data. The B 1 or excitation field may be represented by a mapping showing amplitudes and phases. In this case only the B 1 phase map is used. Next in step 204 a conductivity map is calculated from the B 1 phase map. And finally in step 206 a temperature map is determined using the conductivity map.

Fig. 3 shows a flow diagram which illustrates a method according to a further embodiment of the invention. First in step 300 B 1 field map magnetic resonance data is acquired. Next in step 302 a B 1 amplitude map is determined using the B1 field map magnetic resonance data. In step 304 a permittivity map is calculated from the B 1 amplitude map. Finally in step 306 a temperature map is determined using the permittivity map.

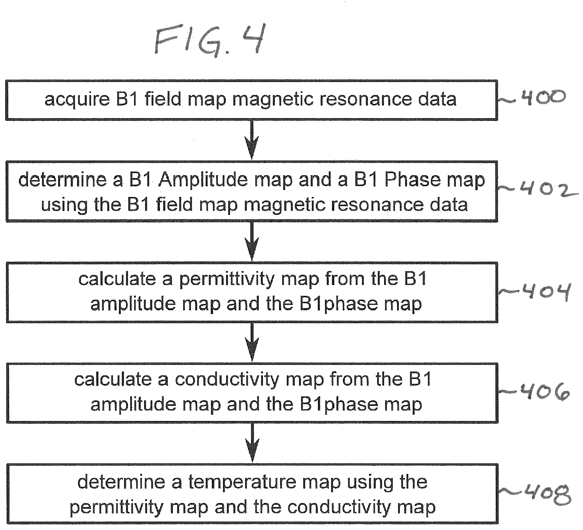

Fig. 4 shows a flow diagram which illustrates a method according to a further embodiment of the invention. First in step 400 B 1 field map magnetic resonance data is acquired. Next in step 402 a B 1 amplitude map and a B 1 phase map are determined using the B 1 field map magnetic resonance data. Next in step 404 a permittivity map is calculated from the B 1 amplitude map and the B 1 phase map. Next in step 406 a conductivity map is calculated from the B 1 amplitude map and the B1 phase map. Steps 404 and 406 may be performed in either order. And finally in step 408 a temperature map is determined using the permittivity map and the conductivity map. Alternative embodiments of this method also exist. Step 404 or step 406 may be omitted. In this case the temperature map is just calculated from the permittivity map or the conductivity map.

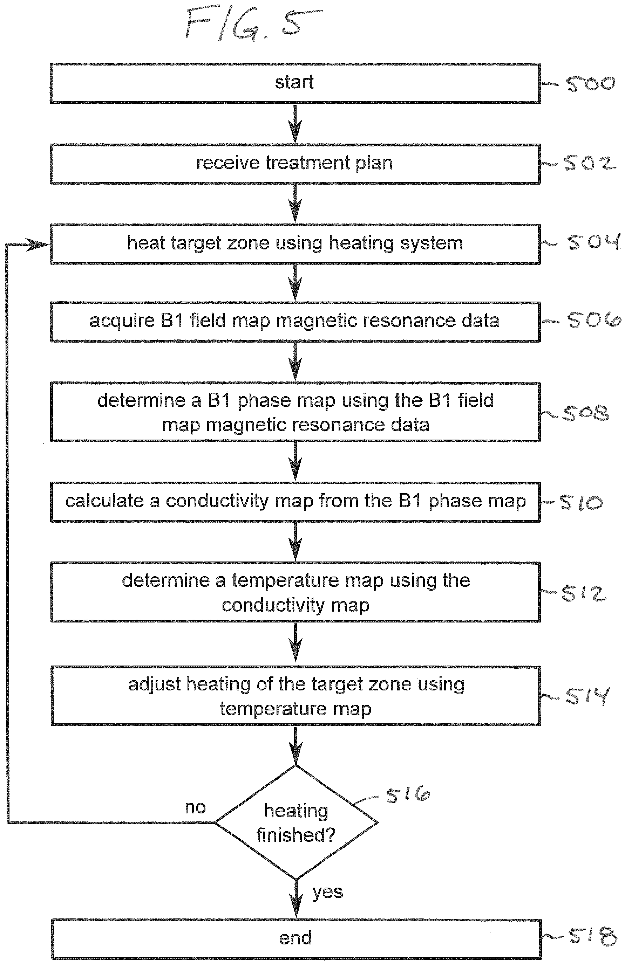

Fig. 5 shows a flow diagram which illustrates a method according to a further embodiment of the invention. In step 500 the method starts. Next in step 502 a treatment plan is received. Next in step 504 a target zone is heated using a heating system. The heating system is controlled in accordance with or using the treatment plan. Next in step 506 a B 1 field map magnetic resonance data is acquired. In step 508 a B 1 phase map using the B 1 field map magnetic resonance data is determined. Next in step 510 a conductivity map is calculated from the B 1 phase map. Then in step 512 a temperature map is determined using the conductivity map. Then in step 514 the heating of the target zone is adjusted using the temperature map. That is to say control commands for adjusting how the target zone is heated using the heating system may be adjusted. It should be noted that steps 506-514 may be performed at the same time as step 504. This is to say while the target zone is being heated by the heating system the magnetic resonance imaging system may acquire the B 1 field map magnetic resonance data and determine a temperature map which is then used to adjust the heating of the target zone while it is in operation. Step 516 is a decision box and the decision is whether the heating is finished or not. If this is not finished then the method returns to step 504 and the method forms a closed control loop. If the heating is finished then step 518 is performed. In step 518 the method ends.

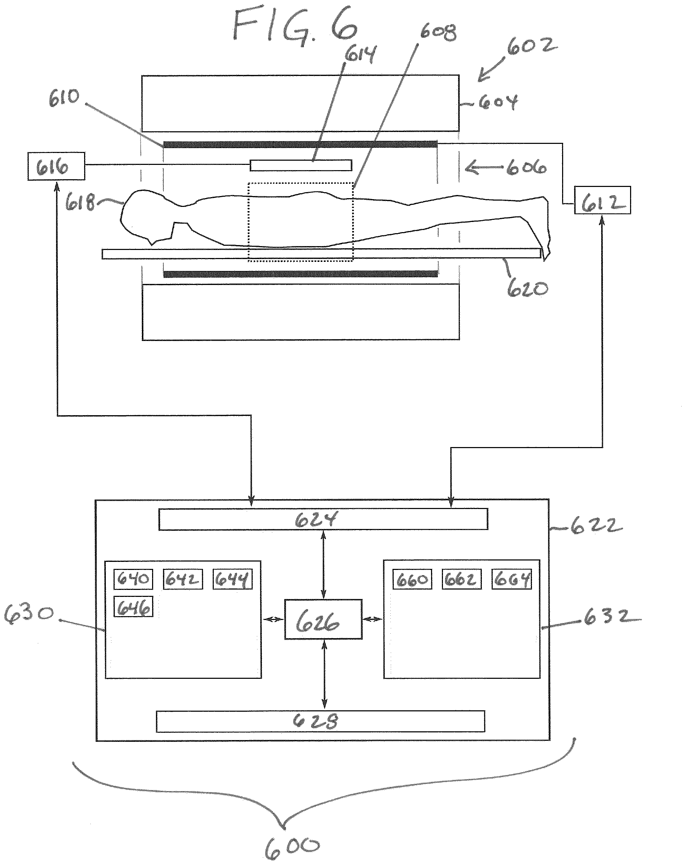

Fig. 6 illustrates a medical apparatus 600 according to an embodiment of the invention. The medical apparatus 600 comprises a magnetic resonance imaging system 602. The magnetic resonance imaging system 602 is shown as comprising a magnet 604. The magnet 604 is a cylindrical type superconducting magnet with a bore 606 through the center of it. The magnet 604 has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore of the cylindrical magnet there is an imaging zone 608 where the magnetic field is strong and uniform enough to perform magnetic resonance imaging.

Also within the bore of the magnet is a magnetic field gradient coil 610 which is used for acquisition of magnetic resonance data to spatially encode magnetic spins within an imaging zone of the magnet. The magnetic field gradient coil 610 is connected to a magnetic field gradient coil power supply 612. The magnetic field gradient coil is representative. Typically magnetic field gradient coils contain three separate sets of coils for spatially encoding in three orthogonal spatial directions. A magnetic field gradient power supply 612 supplies current to the magnetic field gradient coils. The current supplied to the magnetic field coils is controlled as a function of time and may be ramped and/or pulsed.

Adjacent the imaging zone 608 is a radio-frequency coil 614. The radio-frequency coil 614 is connected to a radio-frequency transceiver 616. Also within the bore of the magnet 604 is a subject 618 that is reposing on a subject support 620 and is partially within the imaging zone 608.

Adjacent to the imaging zone 608 is a radio-frequency coil 614 for manipulating the orientations of magnetic spins within the imaging zone 608 and for receiving radio transmissions from spins also within the imaging zone 608. The radio-frequency coil 614 may contain multiple coil elements. The radio-frequency coil 614 may also be referred to as a channel or an antenna. The radio-frequency coil is connected to a radio frequency transceiver 616. The radio-frequency coil 614 and radio frequency transceiver 616 may be replaced by separate transmit and receive coils and a separate transmitter and receiver. It is understood that the radio-frequency coil 614 and the radio-frequency transceiver 616 are representative. The radio-frequency coil 614 is intended to also represent a dedicated transmit antenna and a dedicated receive antenna. Likewise the transceiver 616 may also represent a separate transmitter and a separate receiver.

The magnetic field gradient coil power supply 612 and the radio-frequency transceiver 616 are connected to a hardware interface 624 of a computer system 622. The computer system 622 further comprises a processor 626. The processor 626 is connected to the hardware interface 624. The hardware interface 624 enables the processor 626 to send and receive data and commands to the magnetic resonance imaging system 602. The computer system 622 further comprises a user interface 628, computer storage 630 and computer memory 632.

The computer storage 630 is shown as containing a pulse sequence 640. The pulse sequence 640 contains a set of commands or a timeline which may be translated into a set of commands for operating the magnetic resonance imaging system 602 such that it is able to acquire magnetic resonance data. The computer storage 630 is further shown as containing B 1 field magnetic resonance data. The computer storage 630 is further shown as containing B 1 map data 644. The B1 map data 644 may be any one of the following: B 1 field phase map, a B 1 field amplitude map, or a combination of a B 1 field phase map and a B 1 field amplitude map. The B 1 map data 644 was determined or generated using the B 1 field map magnetic resonance data 642. The computer storage 630 is further shown as containing a temperature map 646. The temperature map 646 was determined using the B 1 map data 644.

The computer memory 632 is shown as containing a control module 660. The control module 660 contains computer executable code which enables the processor 626 to control the operation and function of the medical apparatus 600. For example the control module 660 can use the pulse sequence 640 to acquire the magnetic resonance data 642. The computer memory 632 is further shown as containing B 1 map reconstruction module 662. The B 1 map reconstruction module 662 contains computer executable code which enables the B 1 map reconstruction module 662 to generate the B 1 map data 644 from the B1 field magnetic resonance data 642. The computer memory 632 further contains a temperature map reconstruction module 664 which enables the processor 626 to construct the temperature map 642 from the B 1 map data 644. In some embodiments there may also be a calibration or calibration data which is either used to absolutely calibrate the temperature map 646 or determine a change in temperature.

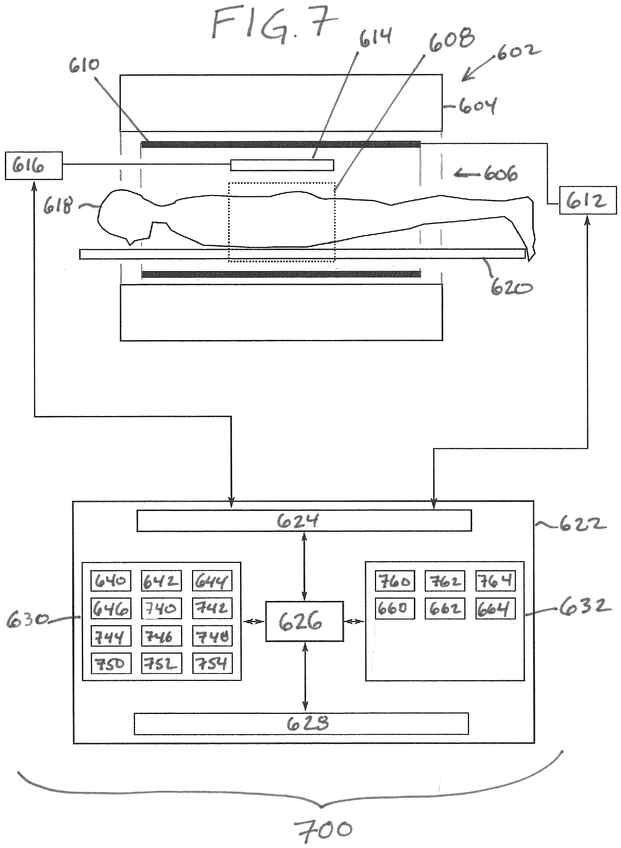

Fig. 7 illustrates a medical apparatus 700 according to a further embodiment of the invention. The embodiment shown in Fig. 7 is similar to that shown in Fig. 6. In addition to the features illustrated in Fig. 6 the medical apparatus 700 in Fig. 7 shows additional features in the computer storage 630 and the computer memory 632. The computer storage 630 is shown as additionally containing a B 1 field phase map 740 and a B 1 field amplitude map 742. Either or both of these maps 740, 742 may be present. The computer storage 630 is also shown as containing a conductivity map 744 and a permittivity map 746. The conductivity map 744 may be approximated using the B 1 field phase map 740 or more accurately calculated using both the B 1 field phase map 740 and the B 1 field amplitude map 742. The computer storage 630 also contains a permittivity map 746 that may be approximately calculated using the B 1 field amplitude map 742 or more accurately calculated using both the B1 field phase map 740 and the B 1 field amplitude map 742. In this embodiment the conductivity map 744 and/or the permittivity map 746 may be present. The computer storage 630 is further shown as containing magnetic resonance data 748. In some embodiments the magnetic resonance data 748 may be identical with the B 1 field magnetic resonance data 642. The computer storage 630 is further shown as containing magnetic resonance image 750 that was generated from the magnetic resonance data 748. The computer storage 630 is further shown as containing a tissue type map 752 that was generated from an image segmentation on the magnetic resonance image 750. The tissue type map 752 may be used to determine an absolute calibration of the B 1 field temperature measurement method. The computer storage 630 further contains a specific absorption ratio map 754. The specific absorption ratio map 754 was calculated at least partially using the temperature map 646.

The computer memory 632 is further shown as containing an image reconstruction module 760. The image reconstruction module contains computer executable code which enables the processor to reconstruct the magnetic resonance image 750 from the magnetic resonance data 748. The computer memory 632 is further shown as containing an image segmentation module 762. The image segmentation module 762 contains computer executable code which enables the processor 626 to reconstruct the tissue type map 752 from the magnetic resonance image 750. The computer memory 632 is further shown as containing a specific absorption ratio map calculation module 764. The specific absorption ratio map calculation module 764 is operable for generating the specific absorption ratio map 754 from the temperature map 646.

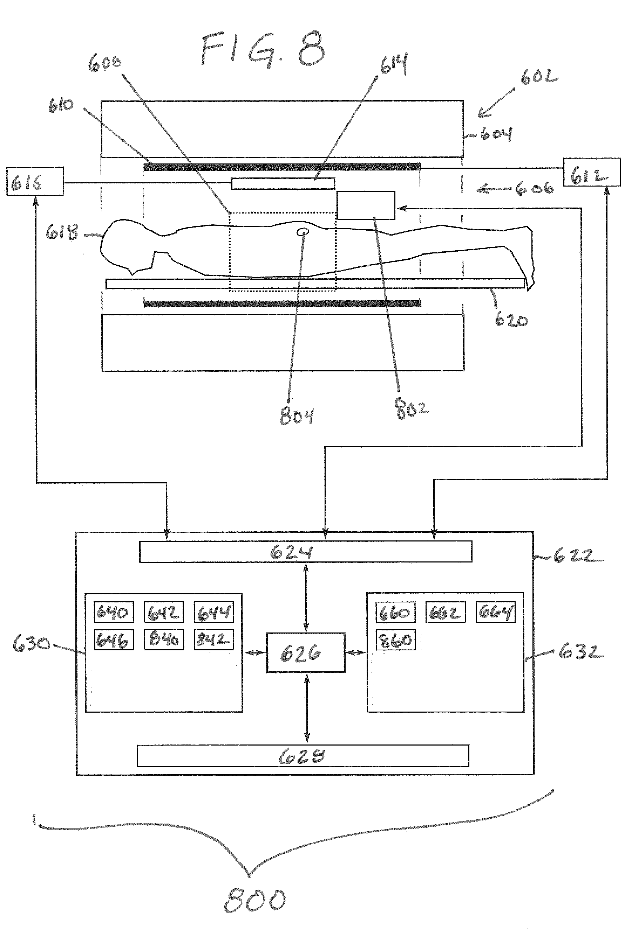

Fig. 8 shows a further embodiment of a medical apparatus 800 according to an embodiment of the invention. In this embodiment the medical apparatus 800 is similar to that shown in Figs. 6 and 7. However, in this case a heating system 802 has been incorporated into the medical apparatus 800. The heating system 800 was connected to the hardware interface 624 of the computer system 622 and is operable for being controlled by the processor 626. The heating system 802 in this embodiment is intended to be generic and may represent any system used for heating a portion of a subject. The heating system 802 may for instance be a high-intensity focused ultrasound system, a radio-frequency heating system, a microwave ablation system, a hyperthermia therapy system, a laser ablation system, and an infrared ablation system. A portion of the subject 618 is indicated as a target zone 804. The heating system 802 is able to controllably heat the target zone 804. The computer storage 630 is shown as containing a treatment plan 840. The treatment plan 840 may be descriptive of the internal structure of the subject 618 and contain data which enables identification or location of the target zone 804. Computer storage 630 further contains a set of heating system control commands 842 that have been generated using the treatment plan 840. The heating system control commands 842 contain commands which enable the processor 626 to control the operation and function of the heating system 802. The computer memory 632 is further shown as containing a heating system control generation module 860. The heating system control generation module 860 contains computer executable code which enables the processor 626 to generate the heating system control commands 842 from the treatment plan 840 and/or the temperature map 646. Using the temperature map 646 enables the processor 626 to form a closed control loop for controlling the operation and function of the heating system 802.

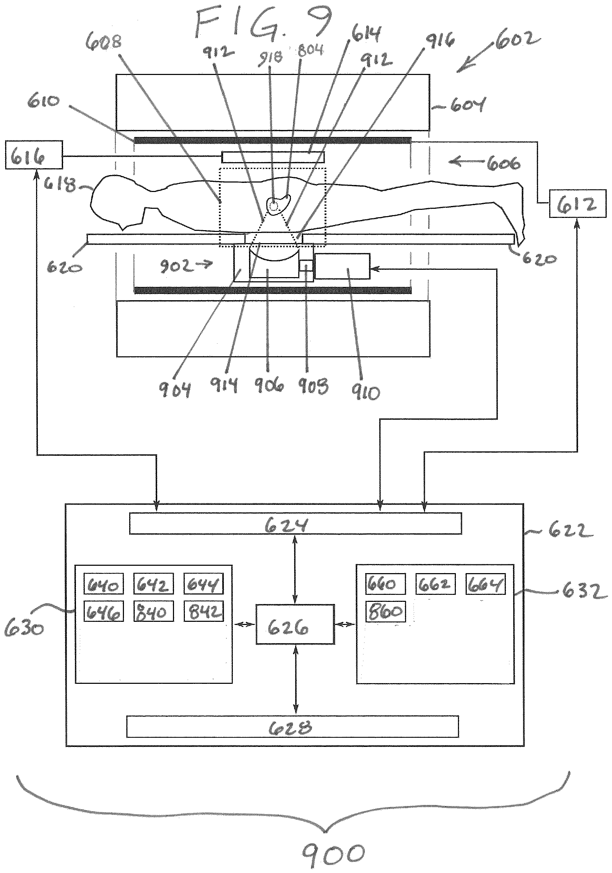

Fig. 9 shows a further embodiment of the medical apparatus 900 according to the invention. In this embodiment the heating system is a high-intensity focused ultrasound system 902. The high-intensity focused ultrasound system comprises a fluid-filled chamber 904. Within the fluid-filled chamber 904 is an ultrasound transducer 906. Although it is not shown in this Fig. the ultrasound transducer 906 may comprise multiple ultrasound transducer elements each capable of generating an individual beam of ultrasound. This may be used to steer the location of a sonication point 918 electronically by controlling the phase and/or amplitude of alternating electrical current supplied to each of the ultrasound transducer elements. The sonication point 918 is operable to be controlled to sonicate the target zone 804.

The ultrasound transducer 906 is connected to a mechanism 908 which allows the ultrasound transducer 906 to be repositioned mechanically. The mechanism 908 is connected to a mechanical actuator 910 which is adapted for actuating the mechanism 908. The mechanical actuator 910 also represents a power supply for supplying electrical power to the ultrasound transducer 906. In some embodiments the power supply may control the phase and/or amplitude of electrical power to individual ultrasound transducer elements. In some embodiments the mechanical actuator/power supply 910 is located outside of the bore 606 of the magnet 604.

The ultrasound transducer 906 generates ultrasound which is shown as following the path 912. The ultrasound 912 goes through the fluid-filled chamber 904 and through an ultrasound window 914. In this embodiment the ultrasound then passes through a gel pad 916. The gel pad is not necessarily present in all embodiments but in this embodiment there is a recess in the subject support 620 for receiving a gel pad 916. The gel pad 916 helps couple ultrasonic power between the transducer 906 and the subject 618. After passing through the gel pad 916 the ultrasound 912 passes through the subject 618 and is focused to a sonication point 918. The sonication point 918 is being focused within a target zone 804. The sonication point 918 may be moved through a combination of mechanically positioning the ultrasonic transducer 906 and electronically steering the position of the sonication point 918 to treat the entire target zone 804.

The high-intensity focused ultrasound system 902 is shown as being also connected to the hardware interference 624 of the computer system 622. The computer system 622 and the contents of its storage 630 and memory 632 are equivalent to that as shown in Fig. 8.

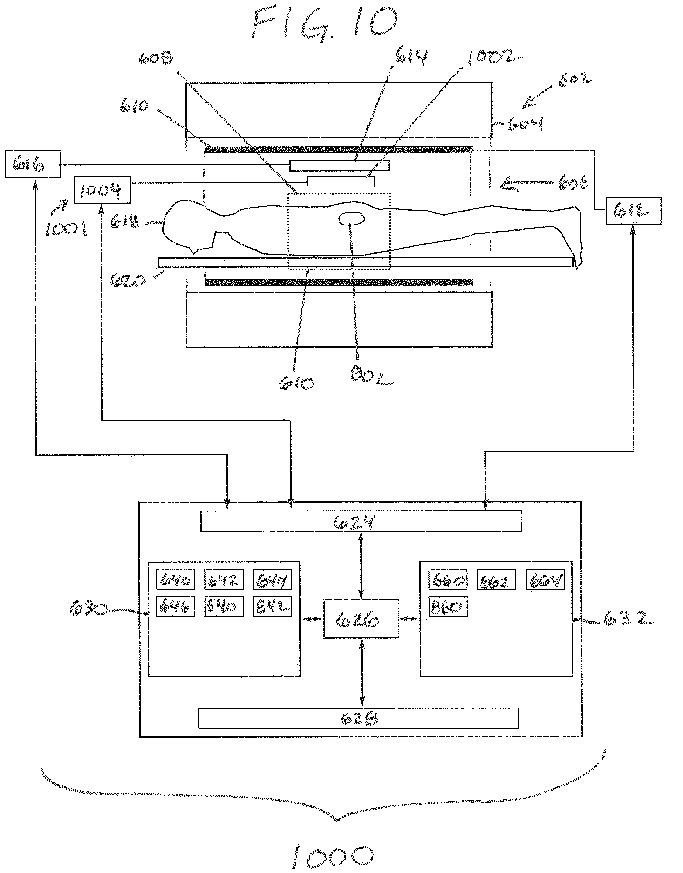

Fig. 10 illustrates a medical apparatus 1000 according to a further embodiment of the invention. In this embodiment the heating system is a radio-frequency heating system 1001. The embodiment shown in Fig. 10 is similar to that shown in Fig. 8. The computer system 622 of Fig. 10 is equivalent to the computer system 622 shown in Figs. 8. The contents of the computer storage 630 and the computer memory 632 are also equivalent to the computer storage 630 and the computer memory 632 as shown in Fig. 8. In the embodiment shown in Fig. 10 a radio-frequency heating system 1001 is used as the heating system. The radio-frequency heating system 1001 comprises an antenna 1002 and a radio-frequency transmitter 1004. The antenna 1002 is in the vicinity of target zone 804. Radio-frequency energy generated by the transmitter 1004 and radiated by the antenna 1002 is used to selectively heat the target zone 804. In this embodiment the radio-frequency transmitter 1004 is shown as being connected to the hardware interface 624. The processor 626 and the contents of the computer storage 630 and the computer memory 632 are used to control the radio-frequency transmitter 1004 in a manner equivalent to the way the high-intensity focused ultrasound system 902 of Fig. 9 is controlled by the processor 626.

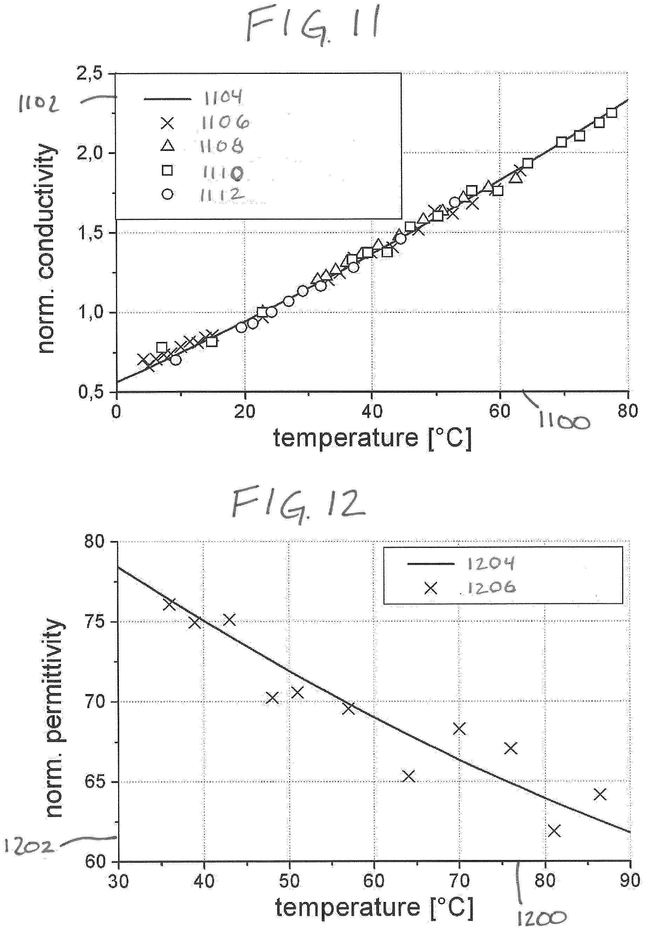

Fig. 11 illustrates how the conductivity can be used to perform a temperature measurement. In Fig. 11 is shown a plot of the temperature 1100 versus the normalized conductivity 1102 of a variety of different samples which are measured using magnet resonance based electric properties tomography measurements. The conductivity measurements are normalized by dividing the conductivity by the conductivity at 23 degrees Celsius. From this plot it can be seen that the normalized conductivity, as measured by electric properties tomography, of water 1106, tomato 1108, apple 1110, and schnitzel 1112 are nearly identical over the range of 0-80 degrees Celsius. The line 1104 indicates values taken from A. Stogryn, "Equations for calculating the dielectric constant of saline water", IEEE Trans. Microwave Theory Tech., MTT-19,733-736, 1971. This indicates that a relatively accurate measurement of the temperature can be performed by measuring the conductivity within a subject. Also the measurements are essentially the same when normalized. This indicates that even if the tissue or material composition of a region of the subject is not known a relative change in temperature can be determined by the change in the normalized conductivity. However, if the tissue or material composition of the region of the subject is known then an absolute measurement can be taken without calibration.

The measurements shown in Fig. 11 were measured using a clinical 1.5T scanner (Achieva, Philips Health care, Best, The Netherlands) using a birdcage head coil. A water phantom, cooked tomato puree, and uncooked apple puree (all 200 cm³) were measured using a 3D balanced FFE sequence (TE/TR = 2.3/4.6 ms, alpha = 45°, voxel size 1×1×3 mm³). A veal sample (Schnitzel, 1 kg) was measured using a 3D TSE sequence (TE/TR = 6/400 ms, voxel size 1×1×3 mm³). The temperature of the different probes was measured using an optical temperature thermometer during MRI imaging. The different probes were heated to the maximum temperature using a microwave oven. During subsequent temperature decay of the probes, conductivity was measured in the MR scanner using the above mentioned sequences with time intervals of 5-15 minutes. An additional series of measurements was acquired with the water phantom after cooling in a refrigerator, following the warming-up to
room temperature. A further measurement was performed with the cooled, uncooked veal sample. The conductivity reconstruction was performed using phase-based EPT. The reconstructed conductivity was averaged over a central 2D ROI (Ø=2 cm).

Fig. 11 illustrates that the method is applicable for a wide range of biological samples and is clearly measurable with EPT. In the investigated example of tissue heating via microwave, this thermal effect completely overrules potential biochemical effects, which could not be identified.

Fig. 12 illustrates how the permittivity can be used to measure the temperature using electric properties tomography. In Fig. 12 a plot of the temperature 1200 versus the normalized permittivity 1202 is shown. The permittivity is normalized such that the permittivity is 80 at room temperature, 25 degrees Celsius. The line 1204 indicates values taken from A. Stogryn, "Equations for calculating the dielectric constant of saline water", IEEE Trans. Microwave Theory Tech., MTT-19,733-736, 1971.

The crosses labeled 1206 show measurements performed using electric properties tomography on water at different temperatures. Figs. 11 and 12 illustrate that the conductivity and/or the permittivity may be used to take accurate measurements of the temperature using a magnetic resonance imaging system.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 600: medical apparatus
- 602: magnetic resonance imaging system
- 604: magnet
- 606: bore of magnet
- 608: imaging zone
- 610: magnetic field gradient coil
- 612: magnetic field gradient coil power supply
- 614: radio frequency coil
- 616: radio frequency transceiver
- 618: subj ect
- 620: subj ect support
- 622: computer system
- 624: hardware interface
- 626: processor
- 628: user interface
- 630: computer storage
- 632: computer memory
- 640: pulse sequence
- 642: B1 field magnetic resonance data
- 644: B 1 map data
- 646: temperature map
- 660: control module
- 662: B 1 map reconstruction module
- 664: temperature map reconstruction module
- 700: medical apparatus
- 740: B 1 flied phase map
- 742: B1 field amplitude map
- 744: conductivity map
- 746: permittivity map
- 748: magnetic resonance data
- 750: magnetic resonance image
- 752: tissue type map
- 754: specific absorption ratio map
- 760: image reconstruction module
- 762: image segmentation module
- 764: specific absorption ratio map calculation module
- 800: medical apparatus
- 802: heating system
- 804: target zone
- 840: treatment plan
- 842: heating system control commands
- 860: heating system control generation module
- 900: medical apparatus
- 902: high intensity focused ultrasound system
- 904: fluid filled chamber
- 906: ultrasound transducer
- 908: mechanism
- 910: mechanical actuator/power supply
- 912: path of ultrasound
- 914: ultrasound window
- 916: gel pad
- 918: sonication point
- 1000: medical apparatus
- 1001: radio-frequency heating system
- 1002: antenna
- 1004: radio-frequency transmitter
- 1100: temperature
- 1102: normalized conductivity
- 1104: H₂O literature
- 1106: H₂O experiment
- 1108: tomato experiment
- 1110: apple experiment
- 1112: schnitzel experiment
- 1200: temperature
- 1202: normalized permittivity
- 1204: H₂O literature
- 1206: H₂O experiment

## Claims

1. A medical apparatus (600, 700, 800, 900, 1000) comprising:
- a magnetic resonance imaging system (602) comprising a magnet (604) with an imaging zone (608) for acquiring magnetic resonance data (642, 748) from a subject (618) from within the imaging zone;
- a memory (632) for storing machine executable instructions (660, 662, 664, 760, 762, 764);
- a processor (626) for controlling the medical apparatus, wherein execution of the instructions causes the processor to:
- acquire (100, 200, 300, 400, 506) B1 field map magnetic resonance data (642) using the magnetic resonance imaging system; and
- determine (102, 206, 306, 408, 512) a temperature map (646) using the B1 field map magnetic resonance data.

2. The medical apparatus of claim 1, wherein execution of the instructions further cause the processor to:
- determine (202) a B 1 field phase map (644) using the B 1 field map magnetic resonance data; and
- calculate (204) a conductivity map (744) from the B 1 field phase map, wherein the temperature map is determined at least partially using the conductivity map.

3. The medical apparatus of claim 1 or 2, wherein execution of the instructions further cause the processor to:
- determine (302) a B 1 amplitude map (644) using the B1 field map magnetic resonance data; and
- calculate (304) a permittivity map (746) from the B 1 field amplitude map, wherein the temperature map is determined at least partially using the permittivity map.

4. The medical apparatus of claim 1, wherein execution of the instructions further cause the processor to:
- determine (402) a B 1 amplitude map (644) and a B 1 phase map (644) using the B 1 field map magnetic resonance data; and
- calculate (406) a conductivity map (744) from the B 1 field phase map and B1 field amplitude map; and
- calculate (404) a permittivity map (746) from the B 1 field phase map and B1 field amplitude map, wherein the temperature map is determined at least partially using the conductivity map and the permittivity map.

5. The medical apparatus of any one of the preceding claims, wherein execution of the instructions further cause the processor to:
- acquire magnetic resonance data (748) using the magnetic resonance imaging system; and
- reconstruct image data (750) from the magnetic resonance data.

6. The medical apparatus of claim 4, wherein execution of the instructions further causes the processor to:
- segment the image data; and
- determine a tissue type map (752) using the segmented image data, wherein the determination of the temperature map is calibrated at least partially using the tissue type map.

7. The medical apparatus of claim 5 or 6, wherein the medical apparatus further comprises a display (628), wherein the method further comprises the step of displaying the temperature map and the image data on the display.

8. The medical apparatus of any one of the preceding claims, wherein execution of the instructions causes the processor to estimate the specific absorption ratio of electromagnetic energy (754) caused by the acquisition of the magnetic resonance data using the temperature map.

9. The medical apparatus of any one of the preceding claims, wherein execution of the instructions further cause the processor to:
- acquire spectroscopic magnetic resonance data; and
- calculate a calibration thermal map using the spectroscopic magnetic resonance data, wherein the determination of the temperature map is calibrated at least partially using the calibration thermal map.

10. The medical apparatus of any one of the preceding claims, wherein the calculation of the temperature map is calibrated at least partially using an assumed calibration thermal map.

11. The medical apparatus of any one of the preceding claims, wherein the medical apparatus further comprises a heating system, wherein execution of the instructions further cause the processor to:
- receive (502) a treatment plan descriptive of the heating of a target zone (804) within the subj ect; and
- heat (504) the target zone using the heating system, wherein the heating system is controlled in accordance with the treatment plan and the temperature map.

12. The medical apparatus of claim 11, wherein execution of the instructions further cause the processor to:
- repeatedly re-acquire the B 1 field map magnetic resonance data and recalculate the temperature map; and
- adjust (514) the heating of the target zone using the re-calculated temperature map.

13. The medical apparatus of claim 11 or 12, wherein the heating system is any one of the following: high intensity focused ultrasound (802, 902), radio-frequency heating system (802, 1001), a microwave ablation system (802), a hyperthermia therapy system (802), a laser ablation system (802), and an infrared ablation system (802).

14. A method of determining a temperature map (646), the method comprising the steps of:
- acquiring (100, 200, 300, 400, 506) B 1 field map magnetic resonance data (642) using a magnetic resonance imaging system; and
- determining (102, 206, 306, 408, 512) the temperature map (646) using the B1 field map magnetic resonance data.

15. A computer program product containing computer executable code (660, 662, 664, 760, 762, 764) for execution by a processor controlling a medical apparatus, wherein the medical apparatus comprises a magnetic resonance imaging system (602), wherein the magnetic resonance imaging system comprises a magnet (604) with an imaging zone (608) for acquiring magnetic resonance data (642, 748) from a subject (618) from within the imaging zone, wherein execution of the instructions cause the processor to:
- acquire (100, 200, 300, 400, 506) B1 field map magnetic resonance data (642) using the magnetic resonance imaging system; and
- determine (102, 206, 306, 408, 512) a temperature map (646) using the B1 field map magnetic resonance data.
